(19) Europäisches Patentamt
European Patent Office
Office européen des brevets

(11) Veröffentlichungsnummer: **0 435 041 A2**

(12) **EUROPÄISCHE PATENTANMELDUNG**

(21) Anmeldenummer: **90123605.9**

(22) Anmeldetag: **08.12.90**

(51) Int. Cl.[5]: **C09B 57/00**, C07D 471/22, C07D 513/22, C07D 495/22

(30) Priorität: **23.12.89 DE 3942893**

(43) Veröffentlichungstag der Anmeldung: **03.07.91 Patentblatt 91/27**

(84) Benannte Vertragsstaaten: **CH DE FR GB LI**

(71) Anmelder: **BAYER AG**
**W-5090 Leverkusen 1 Bayerwerk(DE)**

(72) Erfinder: **Höchstetter, Hans, Dr.**
**Färberstrasse 150**
**W-4000 Düsseldorf(DE)**

(54) **Heterozyklische Verbindungen.**

(57) Heterocyclische Verbindungen der Formel

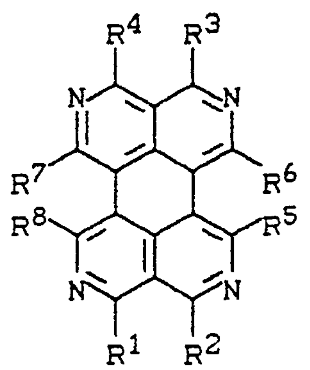

I,

mit den in der Beschreibung angegebenen Substituentenbedeutungen und deren Derivate eignen sich für das Färben und Pigmentieren der verschiedenartigsten Substrate.

EP 0 435 041 A2

## HETEROCYCLISCHE VERBINDUNGEN

Die Erfindung betrifft heterocyclische Verbindungen der Formel

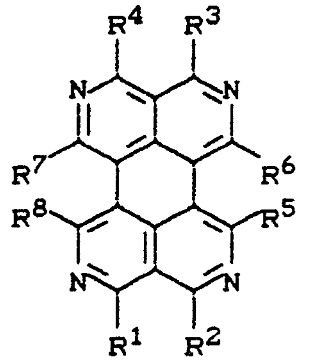

I,

worin

$R^1$ bis $R^8$ = H, Halogen, Alkyl, Cycloalkyl, Aralkyl, Aryl,heterocyclischer Rest, $NR^9R^{10}$, $OR^9$, $SR^9$ wobei

$R^9$, $R^{10}$ = H, Alkyl, Cycloalkyl, Aralkyl, Aryl oder heterocyclischer Rest bedeuten

bzw. die Reste $R^1$, $R^2$ sowie $R^3$, $R^4$, weiterhin $R^5$, $R^6$ und $R^7$, $R^8$ zusammen Teile ankondensierter carbocyclischer oder heterocyclischer 5-, 6- oder 7-gliedriger Ringe bilden können.

Halogen steht vorzugsweise für Cl, Br, F.

Die Reste $R^1$ bis $R^{10}$ können unabhängig voneinander die angegebene Bedeutung haben.

Die Alkyl-, Cycloalkyl-, Aralkyl-, Aryl- und heterocyclischen Reste können übliche Substituenten aufweisen.

Alkyl steht vorzugsweise für $C_1$-$C_{18}$-Alkyl, insbesondere für $C_1$-$C_4$-Alkyl. Cycloalkyl steht vorzugsweise für $C_3$-$C_7$-Cycloalkyl, insbesondere Cyclohexyl und Cyclopentyl. Als Substituenten der Alkyl- und Cycloalkylreste kommen z.B. in Frage: Halogen wie Cl, Br, F, $OCOR^{11}$, $OR^{11}$, $SR^{11}$, $NR^{12}R^{13}$, $OCONR^{12}R^{13}$, $COOR^{11}$, $NR^{12}COR^{11}$, $NR^{12}COOR^{11}$, $CONR^{12}R^{13}$, CN, $SO_2R^{11}$, $COR^{11}$, $SO_2OR^{11}$, $-N=N-R^{14}$, $SO_2NR^{12}R^{13}$.

Besonders bevorzugte Alkyl- und Cycloalkylreste für $R^1$ bis $R^8$ sind solche, die in $\alpha$-Stellung eine carbanionenstabilisierende Gruppe wie CN, $COOR^{11}$, $COR^{11}$, $CONR^{12}R^{13}$, $SO_2R^{11}$ oder $-N=N-R^{14}$ tragen.

Die Reste $R^{11}$ bis $R^{14}$ besitzen die unten angegebenen Bedeutungen.

Aralkyl steht insbesondere für Phenyl- und Naphthyl-$C_1$-$C_4$-Alkyl, wobei die Alkylkohlenstoffatome wie oben für Alkyl beschrieben und die Arylreste wie unten für Aryl beschrieben substituiert sein können.

Aryl steht vorzugsweise für solche carbocyclisch aromatische Reste, die 1, 2, 3 oder 4, insbesondere 1 oder 2, Ringe enthalten wie Phenyl, Diphenylyl oder Naphthyl.

Als heterocyclische Reste stehen vorzugsweise solche heteroaromatische Reste, die 1, 2, 3 oder 4, insbesondere 1 oder 2, fünf-, sechs- oder siebengliedrige Ringe enthalten, von denen mindestens einer 1, 2 oder 3, bevorzugt 1 oder 2 Heteroatome aus der Reihe O, N, S enthält. Als heterocyclische Reste seien beispielhaft genannt:

Pyridyl, Pyrimidyl, Pyrazinyl, Triazinyl, Furoyl, Pyrrolyl, Thiophenyl, Chinolyl, Cumarinyl, Benzofuranyl, Benzimidazolyl, Benzoxazolyl, Dibenzofuranyl, Benzothiophenyl, Dibenzothiophenyl, Indolyl, Carbazolyl, Pyrazolyl, Imidazolyl, Oxazolyl, Isoxazolyl, Thiazolyl, Indazolyl, Benzthiazolyl, Pyridazinyl, Cinnolyl, Chinazolyl, Chinoxalyl, Phthalazinyl, Phthalazindionyl, Phthalimidyl, Chromonyl, Naphtholactamyl, Chinolonyl, ortho-Sulfobenzoesäureimidyl, Maleinimidyl, Naphtharidinyl, Benzimidazolonyl, Benzoxazolonyl, Benzthiazolonyl,Benzthiazothionyl, Chinazolonyl, Chinoxalonyl, Phthalazonyl, Dioxopyrimidinyl, Pyridonyl, Isochinolonyl, Isochinolinyl, Isothiazolyl, Benzisoxazolyl, Benzisothiazolyl, Indazolonyl, Acridinyl, Acridonyl, Chinazolindionyl, Chinoxalindionyl, Benzoxazindionyl, Benzoxazinonyl und Naphthalimidyl.

Die Aryl- und heterocyclischen Reste können beispielsweise durch Halogen wie Chlor, Brom und Fluor, CN, $OR^{11}$, $SR^{11}$, $OCOR^{11}$, $OCONR^{12}R^{13}$, $NR^{12}R^{13}$, $NR^{12}COR^{11}$, $NR^{12}COOR^{11}$, $COR^{11}$, $COOR^{11}$, $CONR^{12}R^{13}$, $SO_2R^{11}$, $SO_2NR^{12}R^{13}$, $-N=N-R^{14}$ oder $R^{15}$ substituiert sein.

$R^{15}$ bezeichnet gegebenenfalls substituiertes Alkyl, vorzugsweise $C_1$-$C_{18}$-Alkyl, insbesondere $C_1$-$C_4$-Alkyl und gegebenenfalls substituiertes Cycloalkyl, vorzugsweise $C_3$-$C_7$-Cycloalkyl, insbesondere Cyclohexyl und Cyclopentyl.

Als Substituenten der Alkyl- und Cycloalkylreste $R^{15}$ können die gleichen stehen, wie sie oben für den Fall $R^1$ bis $R^8$ gleich Alkyl oder Cycloalkyl beschrieben sind.

$R^{11}$, $R^{12}$ und $R^{13}$ bezeichnen Wasserstoff, gegebenenfalls substituiertes Alkyl, insbesondere $C_1$-$C_{18}$-Alkyl, vorzugsweise $C_1$-$C_4$-Alkyl, gegebenenfalls substituiertes Cycloalkyl, insbesondere Cyclohexyl und Cyclopentyl, gegebenenfalls substituiertes Aralkyl, insbesondere Phenyl- und Naphthyl-$C_1$-$C_4$-Alkyl, gegebenenfalls substituiertes Aryl, insbesondere Phenyl und Naphthyl, und einen gegebenenfalls substituierten heterocyclischen Rest, insbesondere den Rest eines fünf- oder sechsgliedrigen heterocyclischen Ringes mit 1, 2 oder 3 Heteroatomen aus der Reihe O, N, S, an den ein Benzolring ankondensiert sein kann.

Die Alkyl- und Cycloalkylreste $R^{11}$, $R^{12}$ und $R^{13}$ können z.B. durch Cl, Br, F, CN, Mono-$C_1$-$C_4$-alkylamino, Di-$C_1$-$C_4$-alkylamino, Phenyl oder Naphthyl, die durch Cl, Br, F, $C_1$-$C_6$-Alkyl und $C_1$-$C_6$-Alkoxy substituiert sein können, oder durch heterocyclische Reste eines 5 - oder 6-gliedrigen heterocyclischen Ringsystems mit 1 oder 2 Heteroatomen aus der Reihe O, N, S, an das ein Benzolring ankondensiert sein kann, substituiert sein.

Auch können $R^{12}$ und $R^{13}$ zusammen unter Einschluß des N-Atoms einen 5- oder 6-gliedrigen heterocyclischen Ring bilden, z.B. einen Morpholin-, Piperidin- oder Phthalimidring. Die Aryl- und Aralkylreste $R^{11}$, $R^{12}$ und $R^{13}$ können beispielsweise durch Cl, Br, F, $C_1$-$C_{18}$-Alkyl, vorzugsweise $C_1$-$C_4$-Alkyl oder durch $C_1$-$C_{18}$-Alkoxy, vorzugsweise $C_1$-$C_4$-Alkoxy, substituiert sein.

$R^{14}$ bezeichnet den Rest einer Kupplungskomponente, vorzugsweise einer Kupplungskomponente aus der Benzol-, Naphthalin-, Acetessigesterarylid-, Pyrazol- oder Pyridonreihe oder einen gegebenenfalls durch Cl, Br, F, $C_1$-$C_{18}$-Alkyl, vorzugsweise $C_1$-$C_4$-Alkyl und $C_1$-$C_{18}$-Alkoxy, vorzugsweise $C_1$-$C_4$-Alkoxy, substituierten Phenylrest.

Falls benachbarte Paare von Resten wie $R^1$-$R^2$, $R^3$-$R^4$, $R^5$-$R^6$ oder $R^7$ -$R^8$ Teile von an das Tetraazaperylensystem ankondensierten fünf-, sechs- oder siebengliedrigen carbo- oder heterocyclischen Ringen sind, so können diese benachbarten Reste-$R^1$-$R^2$- usw. für folgende Verbrückungen stehen:

-S-, -S-S-, 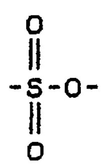, -N=N-, 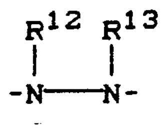, 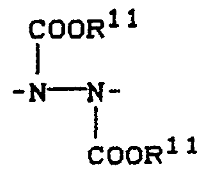,

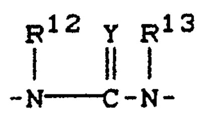, 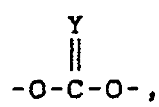, 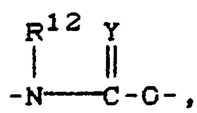, 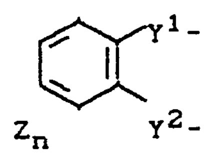,

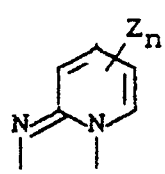, 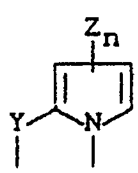, 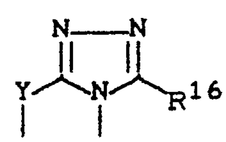, 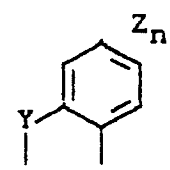,

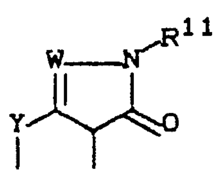, 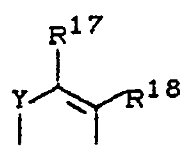, 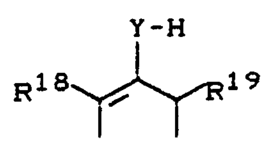

In dieser Zusammenstellung bedeuten:

$R^{11}$, $R^{12}$, $R^{13}$ besitzen die oben beschriebenen Bedeutungen Y, $Y^1$, $Y^2$ = O, S, $NR^{12}$, $NR^{13}$

Z = $OR^{11}$, $NR^{12}R^{13}$, $SR^{11}$, $COOR^{11}$, CN, Br, Cl, F, $CONR^{12}R^{13}$, $SO_2R^{11}$, $SO_2OR^{11}$, $SO_2NR^{12}R^{13}$, $-N{=}N-R^{14}$ $R^{15}$, $OCOR^{11}$, $NR^{12}COR^{11}$, $COR^{11}$.

Der Index n am Substituenten Z kann für 0, 1, 2 und 3 stehen. $R^{14}$ und $R^{15}$ haben die oben angegebenen Bedeutungen.

$R^{16}$ kann für H oder Z stehen,

W kann für =N- oder

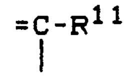

stehen,

$R^{17}$ steht für einen gegebenenfalls substituierten Aryl- oder Hetarylrest, der wie oben für $R^1$ = Aryl und Hetaryl beschrieben substituiert sein kann,

$R^{18}$ = CN, $COOR^{11}$, $COR^{11}$,

$R^{19}$ = CN, $COOR^{11}$, $COR^{11}$, $SO_2R^{11}$, $COR^{11}$, $CONR^{12}R^{13}$.

In $R^{18}$ und $R^{19}$ besitzen $R^{11}$ bis $R^{13}$ die oben beschriebenen Bedeutungen.

Es können ein oder mehrere Ringe an das Tetraazaperylensystem von 1 ankondensiert sein, bevorzugt sind solche Systeme mit zwei oder vier ankondensierten Ringen.

Auch Kombinationen der oben angeführten Verbrückungen sind möglich.

Im Rahmen der Formel I bevorzugte Verbindungen entsprechen der Formel

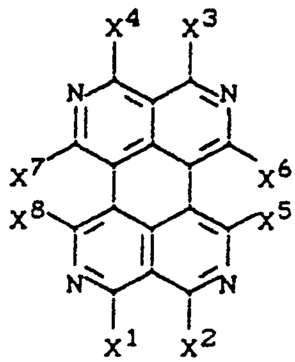

II,

worin $X^1$ bis $X^8$ gleich oder verschieden sein können und für Halogenatome wie Br, Cl und F stehen.

Besonders bevorzugt im Rahmen der Formel II ist die Verbindung IIa, in der $X^1$ bis $x^8$ für Chlor steht.

Weiterhin bevorzugt im Rahmen der Formel I sind Verbindungen der Formel

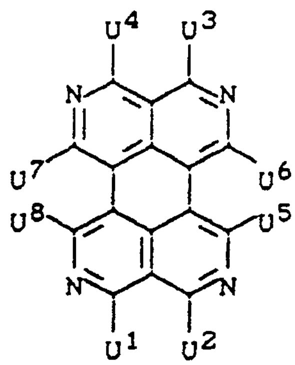

III,

worin $U^1$ bis $U^8$ gleich oder verschieden voneinander sein können und für $OR^9$, $SR^9$ und $NR^9R^{10}$ stehen können, $R^9$ und $R^{10}$ haben die oben angegebenen Bedeutungen.

Besonders bevorzugt im Rahmen der Formel I sind Verbindungen der Formel

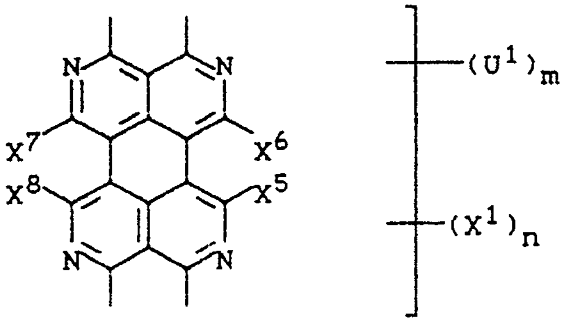

IV,

worin

$U^1$ für gleiche oder verschiedene Reste $OR^9$, $SR^9$, $NR^9R^{10}$ steht; $X^1$ bis $X^8$ gleich oder verschieden sind und für F, Cl, Br stehen, und m und n = 1 - 4 bedeuten, wobei m + n = 4 sind,

Bevorzugt im Rahmen der Formel IV sind Verbindungen der Formel

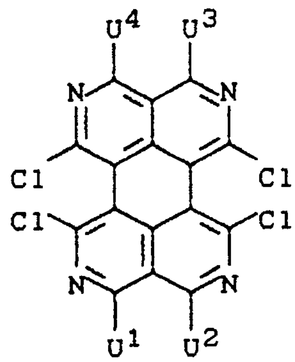

V,

mit den oben angegebenen Bedeutungen für $U^1$ bis $U^4$.

Bevorzugt im Rahmen der Formel V sind Verbindungen Va, in denen $U^1$ bis $U^4$ gleich oder verschieden voneinander sind und für

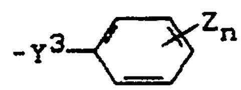

stehen. Z besitzt die oben angegebenen Bedeutungen, der Index n kann für 0, 1, 2 oder 3 stehen. $Y^3$ steht für O, S oder NH, wobei im letzteren Fall die Verbindungen V in verschiedenen tautomeren Formen vorliegen können.

Weiterhin bevorzugt im Rahmen der Formel I sind Verbindungen der Formel

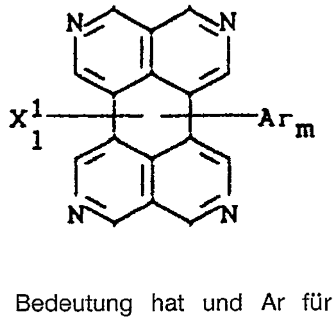

VI,

wobei $X^1$ die obengenannte Bedeutung hat und Ar für einen solchen gegebenenfalls substituierten aromatischen oder heterocyclischen Rest steht, wie er oben für $R^1$ näher beschrieben ist.

Die Indices l und m können Werte zwischen 0 und 8 annehmen, ihre Summe muß 8 betragen.

Benachbarte Gruppen Ar können auch Teile eines einzigen kondensierten aromatischen Systems sein.

Weiterhin bevorzugt im Rahmen der Formel I sind Verbindungen der Formel

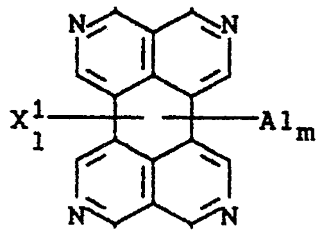

VII,

wobei $X^1$ die obengenannte Bedeutung hat und Al für einen solchen gegebenenfalls substituierten Alkyl-, Cycloalkyl- oder Aralkylrest steht, wie er oben für $R^1$ näher beschrieben ist, steht.

Für die Indices l und m gelten die gleichen Bedingungen wie für Formel VI beschrieben.

Bevorzugt für Al in Formel VII sind solche Alkyl-, Cycloalkyl- und Aralkylreste, die in ∝-Stellung (Verknüpfungsposition zum Pentacyclus) eine carbanionenstabilisierende Gruppe tragen wie CN, $COOR^{11}$, $CONR^{12}R^{13}$, $COR^{11}$, $SO_2R^{11}$ oder $-N=N-R^{14}$, wobei die Reste $R^{11}$ bis $R^{14}$ die oben angegebenen Bedeutungen besitzen.

Weiterhin bevorzugt im Rahmen der Formel I sind Verbindungen der Formel

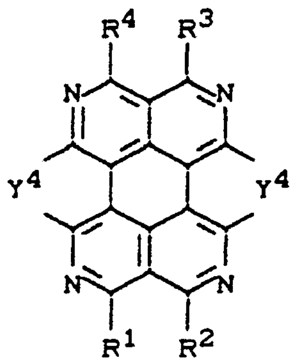

VIII,

worin $R^1$ bis $R^4$ die oben beschriebenen Bedeutungen haben und $-Y^4-$ für -S-, -S-S-, -N=N-,

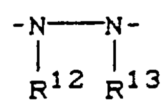

und

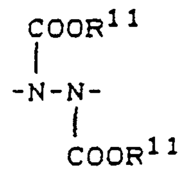

stehen kann. $R^{11}$ bis $R^{14}$ besitzen die oben angegebenen Bedeutungen.

Bevorzugt im Rahmen der Formel VIII sind Verbindungen der Formel

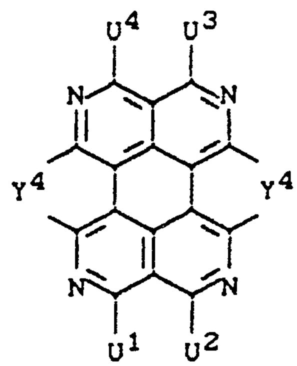

IX,

mit den oben beschriebenen Bedeutungen für $U^1$ bis $U^4$ und $-Y^4-$.

Bevorzugt im Rahmen der Formel IX sind Verbindungen IXa, in denen $-Y^4-$ für -S- steht.

Besonders bevorzugt im Rahmen der Formel VIII sind Verbindungen der Formel

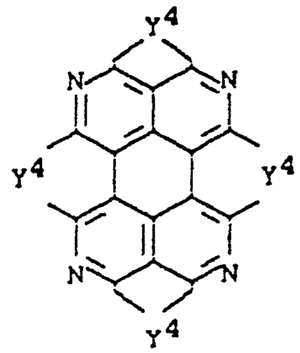

X,

mit den oben beschriebenen Bedeutungen für $-Y^4-$, wobei $-Y^4-$ aber nicht Bestandteil eines Vierrings sein darf.

Weiterhin bevorzugt im Rahmen der Formel I sind Verbindungen der Formel

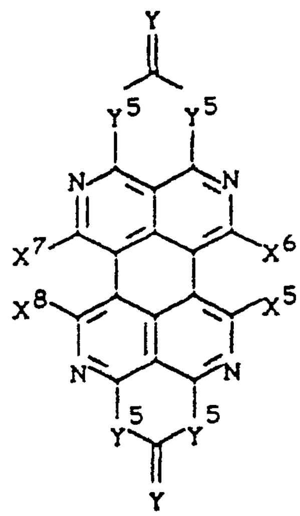

XI,

worin $Y^5$ die Bedeutungen O, $NR^{12}$ und Y und $X^5$ bis $X^8$ die oben beschriebenen Bedeutungen haben.

Bevorzugt im Rahmen der Formel XI sind Verbindungen der Formel XII, in denen $X^5$ bis $X^8$ für Chlor und $Y^5$ für NH stehen.

Weiterhin bevorzugt im Rahmen der Formel I sind Verbindungen der Formel

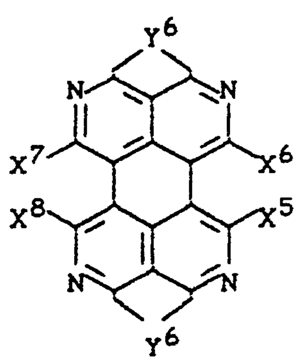

XIII,

worin $X^5$ bis $X^8$ die oben angegebenen Bedeutungen haben und $-Y^6-$ für die nachfolgenden Verbrückungen stehen kann:

(a): 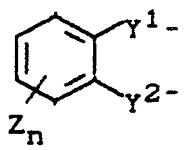 , (b): 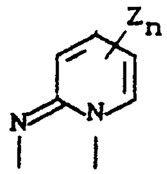 , (c): 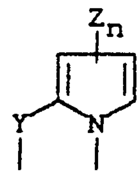

(d): 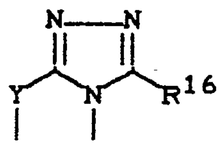 , (e): 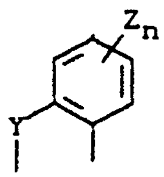 , (f): 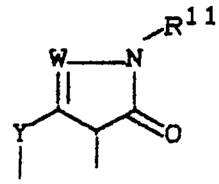

(g): 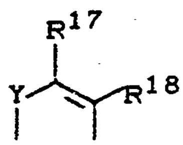 , (h): 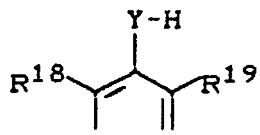 , (i): -S-S-

W, Y, $Y^1$, $Y^2$, $Z_n$, $R^{11}$ und $R^{16}$ bis $R^{19}$ besitzen die oben angegebenen Bedeutungen. Da es sich um unsymmetrische Verbrückungen handelt, kann XIII als Gemisch von zwei Isomeren vorliegen. Ferner können bei gewissen Verbrückungen mehrere tautomere Formen vorliegen.

Bevorzugt im Rahmen der Formel XIII sind Verbindungen der Formel

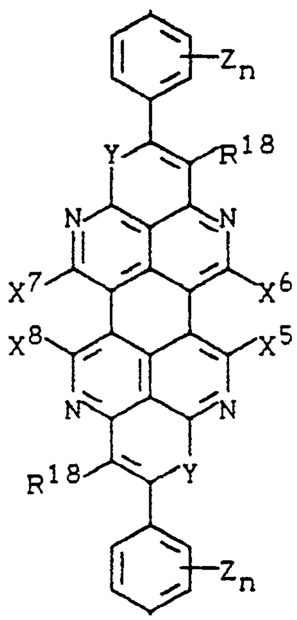

XIVa

bzw.

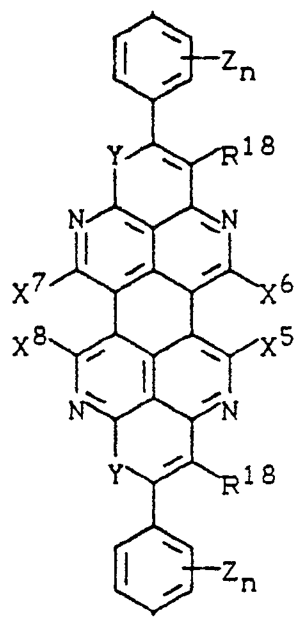

XIVb

mit den oben angegebenen Bedeutungen für $Z_n$, Y, $R^{18}$ und $X^5$ bis $X^8$.

Weiterhin bevorzugt im Rahmen der Formel XIII sind Verbindungen die in einer der möglichen tautomeren Formen den Formeln

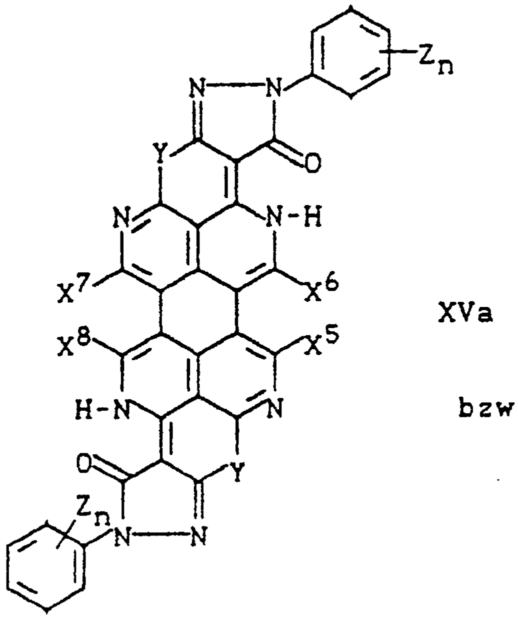

XVa

bzw.

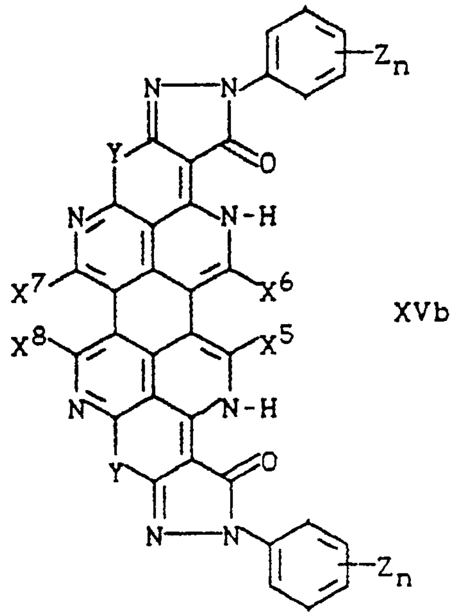

XVb

entsprechen.

Die Substituenten $Z_n$, Y und $X^5$ bis $X^8$ haben die oben beschriebenen Bedeutungen.

Weiterhin bevorzugt im Rahmen der Formel I sind Verbindungen der Formel

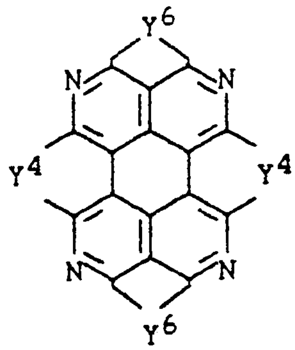

XVI,

mit den oben beschriebenen Bedeutungen für $Y^4$ und $Y^6$.

Die in J. Chem. Soc. 1960, S. 3513 ff. beschriebene Oxidation von

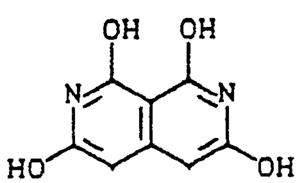

XVII

liefert einen Pentacyclus der vermutlichen Struktur

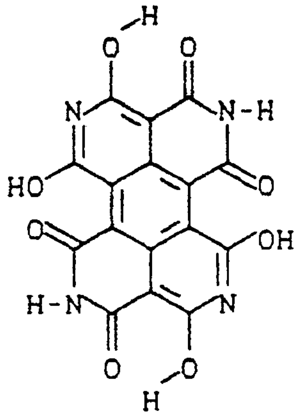

XVIIIa

bzw.

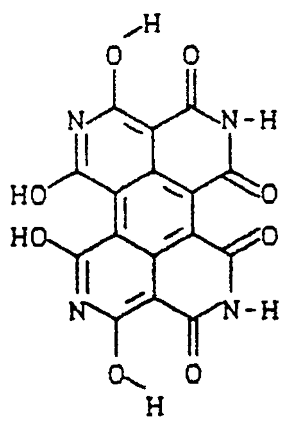

XVIIIb

oder eines der möglichen Tautomeren mit der Symmetrie von XVIIIa ($C_{2h}$) bzw. XVIIIb ($C_{2v}$), z.B.

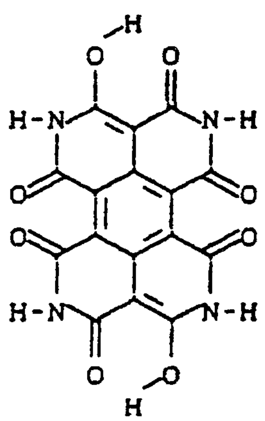

XIX.

Verbindungen des Typs II werden hergestellt, indem man die Verbindung XVIII mit Halogenierungsmitteln wie $PBr_5$, $PCl_5$ oder $POCl_3$ bei Temperaturen zwischen 120° C und 240° C, vorzugsweise zwischen 140° C und 160° C, gegebenenfalls in Gegenwart inerter Lösungsmittel wie Nitrobenzol oder o-Dichlorbenzol umsetzt.

Gegebenenfalls muß die Reaktion in einem Autoklaven unter Druck durchgeführt werden. Die Stöchiometrie muß so berechnet werden, daß vom Halogenierungsmittel unter den Reaktionsbedingungen mindestens 10 Halogenatome pro Molekül XVIII geliefert werden können. Es handelt sich bei dieser Reaktion formal um eine reduktive Halogenierung, die über ein Decahalogenderivat von XVIII läuft. Die Verbindungen des Typs II sind braunorange bis braunrote Feststoffe, die in unpolaren Lösungsmitteln löslich sind.

Verbindungen des Typs III werden erhalten, indem man Verbindungen des Typs II mit Nucleophilen $U^1$-H bis $U^8$-H bei Temperaturen von 20 °C bis 260 °C, vorzugsweise 60 °C bis 150 °C, gegebenenfalls in inerten Lösungsmitteln wie Toluol oder o-Dichlorbenzol, gegebenenfalls in Gegenwart säurebindender Mittel wie Alkalicarbonaten oder tertiären Aminen, umsetzt. Bisweilen kann das Nucleophil U-H selbst säurebindendes Mittel sein. Es können auch die Salze der Nucleophile $U^1$-H bis $U^8$-H eingesetzt werden.

Bei diesen Substitutionsreaktionen zeigen die Halogene $X^1$ bis $X^8$ in II eine deutlich abgestufte Reaktivität` Die Atome $X^1$ bis $X^4$ können in allen Fällen unter deutlich milderen Bedingungen (Temperatur, Druck) ausgetauscht werden als $X^5$ bis $X^8$. Sollen auch diese ausgetauscht werden, muß die Reaktion mit großem Überschuß Nucleophil U-H bei Temperaturen über 200 °C und meist unter Druck durchgeführt werden.

Die Substituenten $X^1$ bis $X^4$ in II können, je nach Stöchiometrie des eingesetzten Nucleophils U-H, auch sukzessive ausgetauscht werden und man kann so Mono- bis Tetrasubstitutionsprodukte IV erhalten, die erforderlichenfalls jeweils isoliert und gegebenenfalls zu weiteren Umsetzungen mit gegebenenfalls anderen Nucleophilen eingesetzt werden können.

Verbindungen des Typs VI können erhalten werden, indem man Verbindungen des Typs II im Sinne einer FriedelCrafts-Acylierung in Gegenwart von mehr als äquimolaren Mengen eines geeigneten Friedel-Crafts-Katalysators wie z.B. $AlCl_3$ mit aromatischen Verbindungen Ar-H umsetzt. Dies erfolgt bei Temperaturen zwischen 100 °C und 250 °C, vorzugsweise zwischen 140 °C und 200 °C, gegebenenfalls kann ein inertes Lösungsmittel zugegen sein,

Je nach Reaktionsbedingungen erhält man Gemische des Typs V1, bei ausreichend energischen Bedingungen können auch Perarylverbindungen VI erhalten werden.

Weiterhin können Verbindungen des Typs VI erhalten werden, indem man Verbindungen des Typs II mit aromatisch-metallorganischen Verbindungen wie Phenyllithium oder Phenylmagnesiumbromid umsetzt.

Verbindungen des Typs VII werden erhalten, indem man Verbindungen des Typs II mit solchen Alkanderivaten G-$CH_2$-$R^{20}$ umsetzt, die Carbanionen bilden und so als Kohlenstoffnucleophile fungieren können. Die Reaktion wird in inerten Lösungsmitteln wie z.B. hochsiedenden, chlorfreien Aromaten durchgeführt, worin zunächst aus Verbindungen des Typs G-$CH_2$-$R^{20}$ das Salz eines Carbanions erzeugt wird. Nach Zugabe des Derivats von II wird dann auf Temperaturen zwischen 50 °C und 200 °C erhitzt.

Der Rest G in G-$CH_2$-$R^{20}$ steht für carbanionenstabilisierende Reste wie CN, $COOR^{11}$, $CONR^{12}R^{13}$, $COR^{11}$, $SO_2R^{11}$ oder $-N=N-R^{14}$, wobei $R^{11}$ bis $R^{14}$ die oben beschriebenen Bedeutungen haben.

$R^{20}$ steht für gegebenenfalls substituiertes Alkyl, insbesondere $C_1$-$C_{18}$-Alkyl, vorzugsweise $C_1$-$C_4$-Alkyl, gegebenenfalls substituiertes Cycloalkyl, insbesondere Cyclohexyl und Cyclopentyl, gegebenenfalls substituiertes Aralkyl, insbesondere Phenyl- und Naphthyl-$C_1$-$C_4$-Alkyl, gegebenenfalls substituiertes Aryl, insbesondere Phenyl und Naphthyl und einen gegebenenfalls substituierten heterocyclischen Rest, insbesondere den Rest eines fünf- oder sechsgliedrigen heterocyclischen Ringes mit 1, 2 oder 3 Heteroatomen aus der Reihe O, N, S, an den gegebenenfalls ein Benzolring ankondensiert sein kann. $R^{20}$ kann durch die oben beschriebenen Substituenten $Z_n$ ($n$ = 0, 1, 2, 3) substituiert sein, wobei Z aber nicht für freie Hydroxyl- und Aminogruppen oder primäre Aminogruppen stehen darf. Ferner kann $R^{20}$ auch die Bedeutung von G annehmen.

Die Carbanionen werden durch Zugabe von Alkalimetallen in geeigneter Form oder von metallorganischen Verbindungen wie Methyl- oder Butyl-Lithium oder von Amidsalzen wie Lithiumdiisopropylamid zu G-$CH_2$-$R^{20}$ erzeugt.

Ferner können die Carbanionenvorläufer G-$CH_2$-$R^{20}$ auch so beschaffen sein, daß die Bedeutung von $R^{20}$ die Möglichkeit von bidenten Kohlenstoffnucleophilen wie G-$CH_2$-$G^1$-$CH_2$-G beinhaltet. $G^1$ steht hier wieder für carbanionenstabilisierende Reste wie

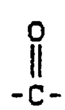

oder $-SO_2-$.

Weiterhin können Verbindungen des Typs VII hergestellt werden, indem man Verbindungen des Typs II mit metallorganischen Alkylderivaten wie Methyl- oder Butyllithium umsetzt.

Verbindungen der Formel IX werden hergestellt, indem man Verbindungen des Typs V mit elementarem Schwefel, Natriumsulfid(hydrat), NaHS, Hydrazin oder einem Hydrazinderivat wie z.B. Hydrazodicarbonsäureester gegebenenfalls in Gegenwart eines hochsiedenden Lösungsmittels wie Ethylenglykol oder Diethylenglykol bei Temperaturen zwischen 100 °C und 250 °C, vorzugsweise zwischen 120 °C und 200 °C,

umsetzt.

Verbindungen der Formel X werden hergestellt, indem man Verbindungen des Typs II mit elementarem Schwefel, Natriumsulfidhydrat, NaHS, Hydrazin oder einem Hydrazinderivat gegebenenfalls in Gegenwart eines hochsiedenden Lösungsmittels bei Temperaturen von 100° C bis 300° C, vorzugsweise zwischen 150° C und 250° C, umsetzt.

Verbindungen des Typs XI werden hergestellt, indem man Verbindungen des Typs II mit Harnstoff oder Harnstoffderivaten wie Monoalkyl-, Monoaryl-, N,N -Dialkyl- oder N,N -Diarylharnstoffen oder Thioharnstoffen in inerten Lösungsmitteln wie Toluol oder Xylolen in Gegenwart säurebindender Mittel wie Alkalicarbonaten oder hochsiedenden tertiären Aminen bei Temperaturen von 100° C bis 200° C, vorzugsweise 120° C bis 140° C, umsetzt. Weiterhin kann II auch in Carbonatschmelzen zu Verbindungen des Typs XI umgesetzt werden.

Verbindungen des Typs XIII werden hergestellt, indem man Verbindungen des Typs II mit folgenden bidenten oder ambidenten Nucleophilen umsetzt:

(a): 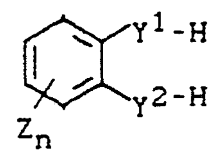 : gegebenenfalls substituierte o-Aminothiophenole, o-Phenylendiamine, o-Aminophenole, Brenzkatechine

(b): 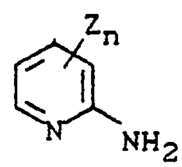 : gegebenenfalls substituierte 2-Aminopyridine

(c): 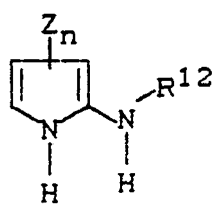 : gegebenenfalls substituierte α-Aminopyrrole

(d): 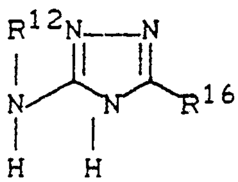 : gegebenenfalls substituierte 3-Amino-1,2,4-triazolderivate

(f): 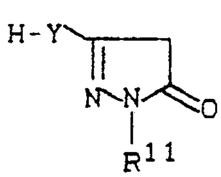 , 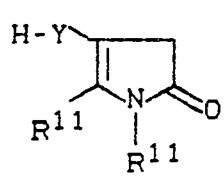 : gegebenenfalls substituierte 3-Amino- oder 3-Hydroxypyrazolone

bzw. 4-Hydroxy- oder 4-Aminopyrrolone, die noch eine C-H-acide Methylengruppe tragen.

(g): 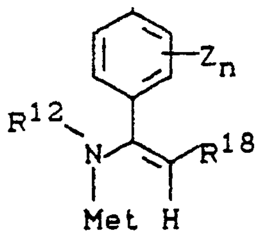 , 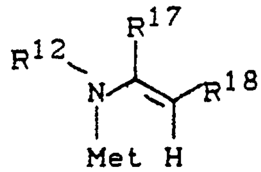 :

Alkalimetallsalze von gegebenenfalls substituierten Aminozimtnitrilen, Aminozimtsäureestern, Aminocrotonitrilen oder Aminocrotonsäureestern in Gegenwart eines weiteren säurebindenden Salzes. Zweckmäßigerweise verwendet man z.B. direkt die bei der Synthese von Aminozimtsäurenitrilen anfallenden Dinatriumsalze (E. v. Meyer, J.pr.Ch. [2] 52, 110).

(h): 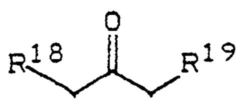 : Glutarnitrilderivate, Glutaresterderivate, Acetondicarbonsäureesterderivate

$$R^{11}-\overset{\overset{O}{||}}{C}-CH_2-\overset{\overset{O}{||}}{C}-CH_2-\overset{\overset{O}{||}}{C}-R^{11}$$ : Diaroyl- oder Diacylacetonderivate

Die Umsetzungen erfolgen in hochsiedenden Lösungsmitteln wie Toluolen, Xylolen oder o-Dichlorbenzol gegebenenfalls in Gegenwart von säurebindenden Mitteln wie Alkalicarbonaten oder tertiären Aminen bei Temperaturen zwischen 100° C und 250° C, vorzugsweise 140° C bis 200° C.

Verbindungen der Formel XIII, in denen $Y^6$ für -S-S-steht, werden hergestellt, indem man Verbindungen des Typs II mit Natriumhydrogensulfidlösung in wassermischbaren Lösungsmitteln wie Alkoholen oder DMF in Gegenwart von Sauerstoff bei Temperaturen zwischen 50° C und 150° C, vorzugsweise zwischen 80° C und 100° C, umsetzt.

Verbindungen des Typs XIII mit der Verbrückung (e) werden hergestellt, indem man Verbindungen des Typs XIII mit der Verbrückung (a), in der einer der Reste $Y^1$ oder $Y^2$ für Schwefel steht, in hochsiedenden inerten Lösungsmitteln wie Biarylen oder Diphenylethern auf Temperaturen zwischen 200° C und 300° C erhitzt, wobei zwei Schwefelatome eliminiert werden.

Weiterhin können Verbindungen XIII (e) hergestellt werden, indem man Verbindungen des Typs IV mit n = 1 und

$U^1_m$ = $-Y^3-$ 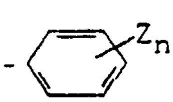 ,

m = 1 auf Temperaturen über 110° C erhitzt, gegebenenfalls in Gegenwart eines geeigneten Katalysators wie $FeCl_3$ und/oder eines säurebindenden Mittels. $Y^3$ und $Z_n$ besitzen die bei Formel Va beschriebenen Bedeutungen.

Verbindungen des Typs XVI werden hergestellt, indem man Verbindungen des Typs XIII mit elementarem Schwefel, Natriumsulfidhydrat, NaHS, Hydrazin oder Hydrazinderivaten gegebenenfalls in hochsiedenden Lösungsmitteln wie Ethylenglykol oder Diethylenglykol gegebenenfalls in Gegenwart säurebindender Mittel bei Temperaturen zwischen 150° C und 300° C, vorzugsweise zwischen 200° C und 250° C, umsetzt. In manchen Fällen muß unter erhöhtem Druck gearbeitet werden,

Substanzen der Formel II stellen wertvolle Zwischenprodukte zur Herstellung von Farbstoffen, Pigmenten, Fotoleitersubstanzen oder von Substanzen für organische Leiter dar.

Die Substanzen vom Typ V stellen lösliche Farbstoffe zum Massefärben von Kunststoffen in gelben bis violetten Tönen dar. Als Kunststoffe seien z.B. Polystyrol, Polycarbonate, Polyurethane, Polyamide oder

Polyolefine genannt. Die Färbungen zeichnen sich durch hohe Brillanz und gute Temperaturstabilität aus.

Substanzen der Formel VI stellen blaue bis grüne Pigmente dar, solche der Formel IV gelbe bis rote Pigmente oder Farbstoffe , solche der Formeln XI und XIII rotviolette bis blaue Pigmente und solche der Formel XVI blaue bis grüne Pigmente.

Die Verbindungen der Formeln VI, IX, XI, XIII, XVI fallen in einer für die Pigmentanwendung geeigneten Form an oder können durch an sich bekannte Nachbehandlungsverfahren in die geeignete Form überführt werden, z.B. durch Lösen oder Quellen in starken anorganischen Säuren wie Schwefelsäure und Austragen auf Eis. Die Feinverteilung kann auch durch Mahlen mit oder ohne Mahlhilfsstoffen wie anorganischen Salzen oder Sand, gegebenenfalls in Anwesenheit von Lösungsmitteln wie Toluol, Xylol, Dichlorbenzol oder N-Methylpyrrolidon erzielt werden. Farbstärke und Transparenz des Pigments können durch Variation der Nachbehandlung beeinflußt werden.

Die Verbindungen der Formel VI, IX, XI, XIII, XVI eignen sich aufgrund ihrer Licht- und Migrationsechtheit für die verschiedensten Pigmentapplikationen. So können sie zur Herstellung von sehr echt pigmentierten Systemen in Mischung mit anderen Stoffen, Zubereitungen, Anstrichmitteln, Druckfarben, gefärbtem Papier und gefärbten makromolekularen Stoffen verwendet werden. Unter Mischung mit anderen Stoffen können z.B. solche mit anorganischen Weißpigmenten wie Titandioxid (Rutil) oder mit Zement verstanden werden. Zubereitungen sind z.B. Flushpasten mit organischen Flüssigkeiten oder Teige und Feinteige mit Wasser, Dispergiermitteln und gegebenenfalls Konservierungsmitteln. Die Bezeichnung Anstrichmittel steht z.B. für physikalisch und oxidativ trocknende Lacke, Einbrennlacke, Reaktionslacke, Zweikomponentenlacke, Dispersionsfarben für wetterfeste Überzüge und Leimfarben.

Unter Druckfarben sind solche für den Papier-, Textil- und Blechdruck zu verstehen. Die makromolekularen Stoffe können natürlichen Ursprungs sein wie Kautschuk, durch chemische Modifikation erhalten werden wie Acetylcellulose, Cellulosebutyrat oder Viskose oder synthetisch erzeugt werden wie Polymerisate, Polyadditionsprodukte und Polykondensate. Genannt seien plastische Massen wie Polyvinylchlorid, Polyvinylacetat, Polyvinylpropionat, Polyolefine, z.B. Polyethylen oder Polyamide, Superpolyamide, Polymerisate und Mischpolymerisate aus Acrylester, Methacrylestern, Acrylnitril, Acrylamid, Butadien, Styrol sowie Polyurethane und Polycarbonate. Die mit den beanspruchten Produkten pigmentierten Stoffe können auch in beliebiger Form vorliegen.

Beispiel 1

40 g der Verbindung XVIII werden mit 240 g $PCl_5$ gemischt und auf einem Ölbad 24 Stunden lang bei 150 °C bis 160 °C Badtemperatur gerührt. Im Verlauf der Reaktion werden nach und nach ca. 60 ml $POCl_3$ abdestilliert, nach 24 Stunden wird das entstandene $POCl_3$ möglichst weitgehend abdestilliert. Der orangefarbene, feste Rückstand, der noch überschüssiges $PCl_5$ enthält, wird auf 2 1 Eiswasser ausgetragen, abgesaugt und im Vakuum getrocknet. Man erhält 52 g einer Verbindung der Formel

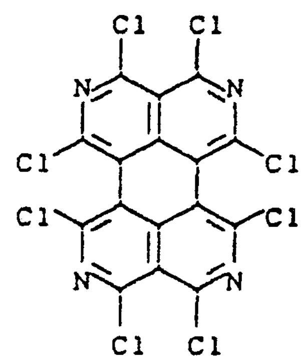

die nötigenfalls durch Rekristallisation aus Nitrobenzol unter Zusatz von $PCl_5$ weiter gereinigt werden kann.

| | |
|---|---|
| IR-Daten: | 1535, 1292, 1240, 1183, 893 $cm^{-1}$ |
| UV VIS-Daten: | $\lambda$ = 452, 480 nm (19 300) |
| $^{13}$C-NMR-Daten (Solid) : | $\delta$ = 117,6, 130,11, 132,3, 145,6, 155,3 ppm. |

Beispiel 2

5,0 g der Verbindung aus Beispiel 1 werden zusammen mit 4,6 g Phenol und 5,7 g Kaliumcarbonat in

10 ml o-Dichlorbenzol 1,5 Stunden lang bei 140° C bis 150° C gerührt. Nach Abkühlen saugt man ab, schlämmt den Nutschkuchen in Wasser an und saugt erneut ab. Man erhält 6,1 g eines leuchtend gelben Kristallpulvers der Formel

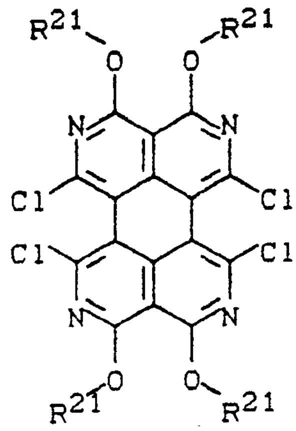

$R^{21}$ = 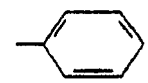

IR-Daten: 3080, 3050, 1570, 1520, 1490, 1390, 1290, 1240, 1205, 940, 810, 750, 690 $cm^{-1}$.
UV/VIS-Daten: $\lambda_{max}$ = 465, 440 nm ($\epsilon$ = 24 600)

Durch ähnliches Vorgehen wie oben in Beispiel 2 lassen sich die in nachfolgender Tabelle aufgeführten Farbstoffe gewinnen.

| Beispiel Nummer | $R^{21}$ | UV/VIS-Daten |
|---|---|---|
| 3 | 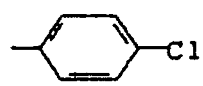 | λ = 466, 440 nm |
| 4 | 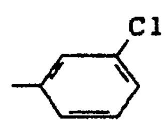 | λ = 465, 435 nm |
| 5 | 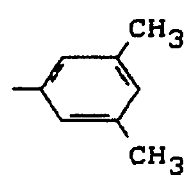 | λ = 468, 441 nm<br>ε = 24 150 |
| 6 | $-CH_3$ | λ = 452, 427 nm |
| 7 | 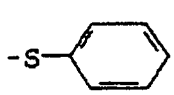 | λ = 536 nm |

Beispiel 8

11,0 g der Verbindung aus Beispiel 1 werden zusammen mit 8,5 g Triethylamin und 3,0 g α-Aminopyridin in 100 ml Toluol 40 Minuten lang am Rückfluß gekocht. Nach Abkühlen wird abgesaugt und mit reichlich Ethanol gewaschen. Man erhält 10 g eines rotorangen Farbstoffs der Formel

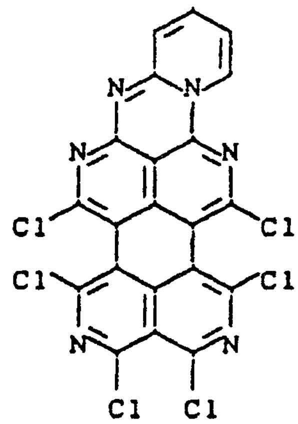

IR-Daten: 1615, 1590, 1490, 1445, 1360, 1330, 1230, 1190, 1155, 1090 $cm^{-1}$
UV VIS-Daten: $\lambda_{max}$ = 547 nm (c = 29 000)

Beispiel 9

3,0 g der Verbindung aus Beispiel 1 werden in 20 ml Anilin 90 Minuten lang bei 150 bis 160° C gerührt. Danach wird abgekühlt, in Ethanol eingerührt und abgesaugt. Der Nutschkuchen wird in Wasser angerührt, erneut abgesaugt und getrocknet. Man erhält 3,5 g einer Verbindung, die in einer ihrer tautomeren Formen der Formel

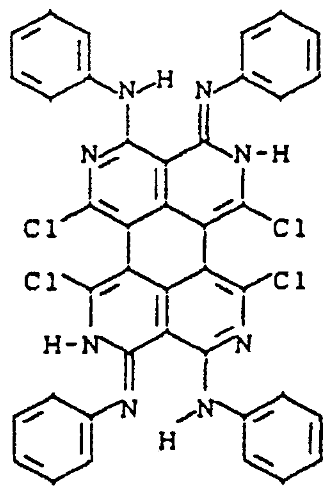

entspricht.
IR-Daten: 1570, 1500, 1450, 1325, 1190, 940 $cm^{-1}$
UV VIS-Daten: $\lambda_{max}$ = 547 nm ($\epsilon$ = 39 300)

Beispiel 10

10 g der Verbindung aus Beispiel 1 werden zusammen mit 5,2 g o-Aminothiophenol und 8,4 g Triethylamin in 100 ml Toluol 4 Stunden lang am Rückfluß gekocht. Nach Abkühlen wird abgesaugt, der Nutschkuchen wird mit Wasser und Methanol gewaschen. Man erhält 10 g eines rotvioletten Pigments der Formel

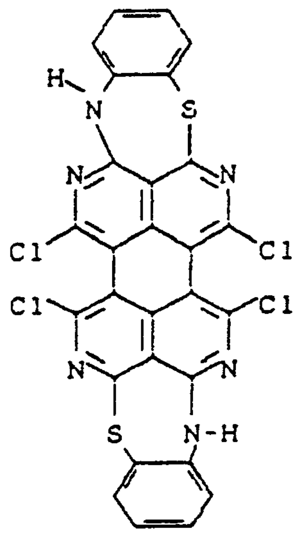

bzw. seines $C_{2v}$-Isomeren.

IR-Daten: 1555, 1485, 1430, 1325, 1278, 1189, 760 $cm^{-1}$

UV VIS-Daten: $\lambda_{max}$ = 560 nm ($\epsilon$ = 20 200)

Beispiel 11

10 g der Verbindung aus Beispiel 10 werden in 100 ml o-Dichlorbenzol 5 Stunden lang am Rückfluß gekocht. Man erhält 8 g eines violetten Pigments der Formel

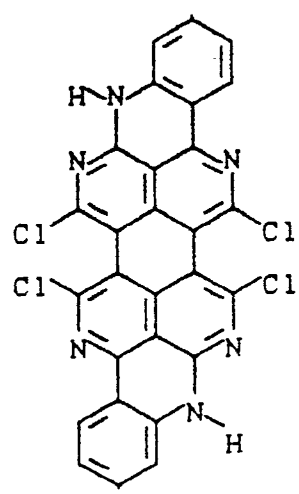

bzw. eines $C_{2v}$-Isomeren bzw. eines Gemisches der beiden Isomeren.

Beispiele 12. 13

Verfährt man nach den Angaben des Beispiels 10 und setzt statt o-Aminothiophenol o-Aminophenol (Beispiel 12) oder o-Phenylendiamin (Beispiel 13) ein, so erhält man rotviolette (Beispiel 13, $\lambda_{max}$ = 532, 502 nm) bzw. orangerote (Beispiel 12, $\lambda$ = 528 nm) Pigmente einer Struktur, die der in Beispiel 10 gezeigten analog ist.

Beispiel 14

Zu 2,0 g der Verbindung aus Beispiel 1 in 20 ml Diethylenglykol werden 5 ml einer 30 %igen wäßrigen NaHS-Lösung zugetropft. Danach wird 90 Minuten lang bei 120° C gerührt. Der Ansatz wird auf 100 ml Wasser ausgetragen, mit HCl angesäuert und abgesaugt. Man erhält 1,9 g eines löslichen, violetten

Farbstoffs der Formel

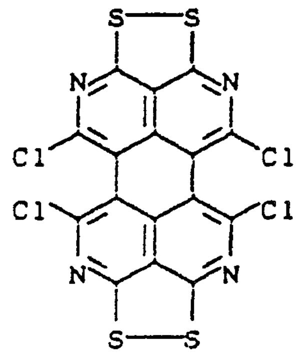

MS-Daten: $m^+ = 516$ ($^{35}Cl_4$)
UV VIS-Daten: $\lambda_{max} = 557, 519$ nm

Beispiel 15

13,2 g der Verbindung aus Beispiel 1, 13,2 g $Na_2S$ * $3H_2O$ und 53 g Schwefel werden gründlich vermischt, auf 250° C (Innentemperatur) erhitzt und 2 Stunden lang bei dieser Temperatur gerührt. Nach dieser Zeit läßt man auf 100° C Innentemperatur abkühlen, setzt 100 ml Toluol zu und gießt dieses Gemisch in 300 ml heißes Toluol. Es wird heiß abgesaugt. Der Nutschkuchen wird zermörsert, nochmals in 300 ml Toluol aufgekocht und abgesaugt.

Der Nutschkuchen wird in 400 ml Wasser angeschlämmt, 12 Stunden lang heftig unter Sauerstoffzutritt gerührt. Nach Absaugen, Waschen mit Wasser und Trocknen erhält man 11 g eines grünen Pigments der Formel

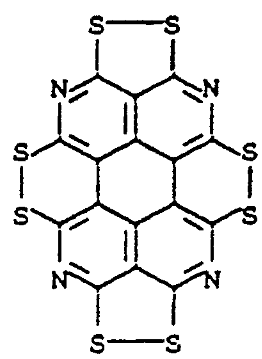

MS: $m^+ = 504$ (100 %, $S_8$-Masse)
IR-Daten: 1580, 1545, 1480, 1376, 1350, 1310, 1230, 1192, 890 $cm^{-1}$

Beispiel 16

5 g der Verbindung aus Beispiel 1, 9 ml Triethylamin und 1,7 g Harnstoff werden in 50 ml Xylol 5 Stunden lang bei 150° C gerührt. Es wird abgesaugt, mit Methanol und Wasser gewaschen und getrocknet. Man erhält 3,5 g eines rotvioletten Pigments der Formel

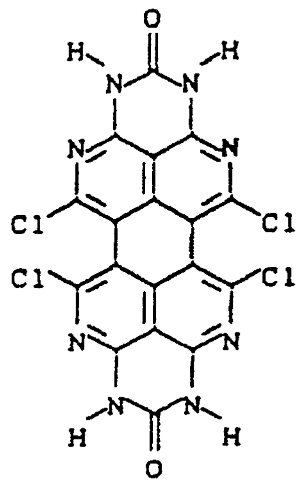

UV VIS-Daten: $\lambda_{max}$ = 545 nm

Beispiele 17 bis 19

Man verfährt wie in Beispiel 16 beschrieben, setzt jedoch statt Harnstoff die substituierten Vertreter N,N'-Dimethylharnstoff, Phenylharnstoff und N,N'-Diphenylharnstoff ein. Man isoliert jeweils rotviolette Pigmente einer zu Beispiel 16 analogen Struktur.

Beispiel 20

3 g der Verbindung aus Beispiel 1 und 7 g eines aus Benzonitril, Acetonitril und Natrium nach J. Pr. Ch. [2] 52, 110 hergestellten Dinatriumsalzes der Verbindung

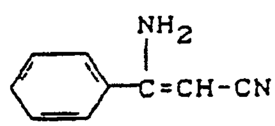

XX

werden 15 Stunden lang in 30 ml Toluol am Rückfluß gekocht. Nach Zugabe von 5 ml Eisessig wird abgesaugt und mit Wasser und Methanol gewaschen. Man erhält 1,3 g eines rotvioletten Pigments der Formel

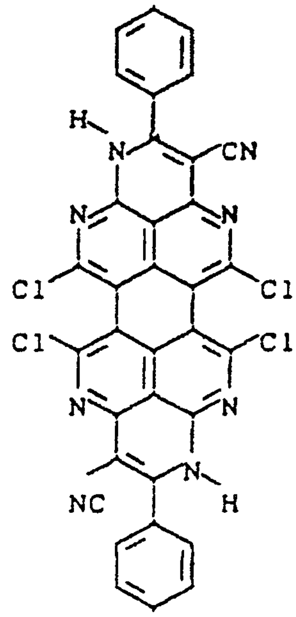

bzw.

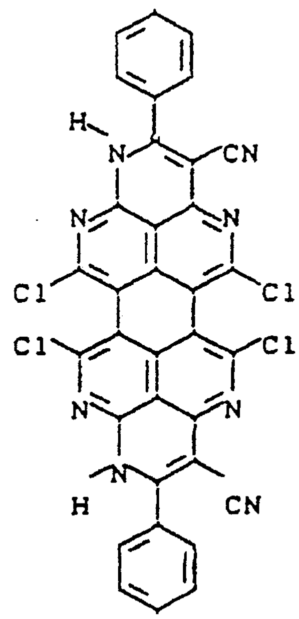

bzw. eines Gemisches der möglichen Isomeren. IR-Daten: 2230, 1600, 1510, 1320, 1190, 700 $cm^{-1}$.

Beispiel 21

2 g der Verbindung aus Beispiel 1 werden zusammen mit 1,6 g des Pyrazolons

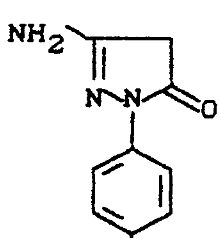

XXI

und 3 ml Triethylamin in 40 ml Toluol 4 Stunden lang am Rückfluß gekocht. Dann wird abgesaugt, der Nutschkuchen wird mit Wasser und Methanol gewaschen. Man erhält 2,4 g eines rotvioletten Pigments der Formel

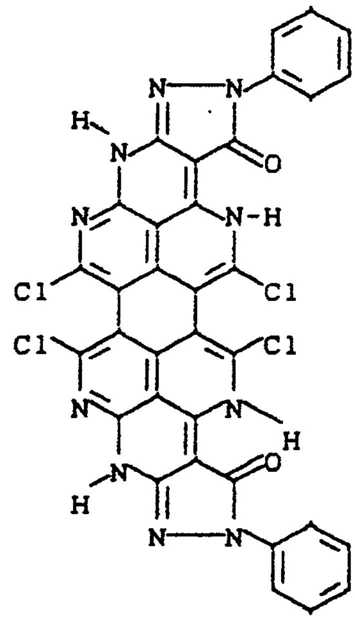

bzw.

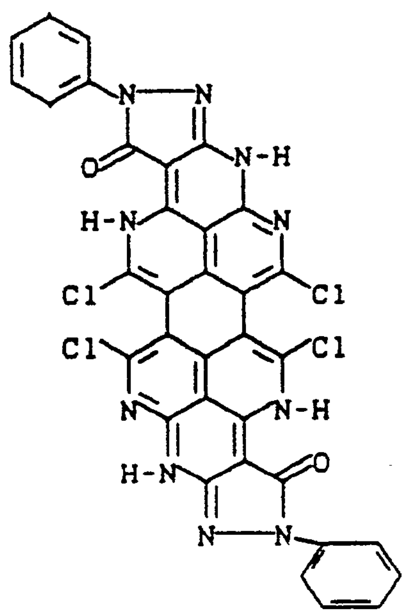

bzw. eines Gemischs der möglichen Isomeren.

Beispiel 22

3 g der Verbindung aus Beispiel 1 werden in 30 ml Hydrazinhydrat 1 Stunde lang bei 80° C gerührt. Danach wird abgesaugt und mit Wasser gewaschen. Man erhält 2,2 g eines braunroten Pigments der vermutlichen Struktur

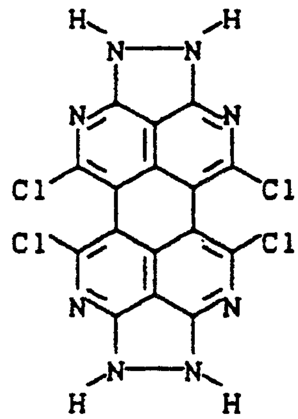

Beispiel 23 (Anwendungsbeispiel)

4 g feingemahlenes Pigment gemäß Beispiel 15 werden in 92 g eines Einbrennlackes folgender Zusammensetzung dispergiert:

33 % Alkydharz
15 % Melaminharz
5 % Glykolmonomethylether
34 % Xylol
13 % Butanol.

Als Alkydharze kommen Produkte auf Basis synthetischer und pflanzlicher Fettsäuren wie Kokosöl, Rizinusöl, Rizinenöl, Leinöl u.a. in Frage. Anstelle von Melaminharzen können Harnstoffharze verwendet werden. Nach erfolgter Dispergierung wird der pigmentierte Lack auf Papier-, Glas-, Kunststoff- oder Metall-Folien aufgetragen und 30 Minuten bei 130 °C eingebrannt. Die Lackierungen besitzen sehr gute Licht- und Wetterbeständigkeit sowie gute Überlackierechtheit.

Dieser Einbrennlack wird auf weißes Papier aufgestrichen, bei 130 °C eingebrannt und zeigt einen grünen Farbton.

## Ansprüche

1. Heterocyclische Verbindungen der Formel

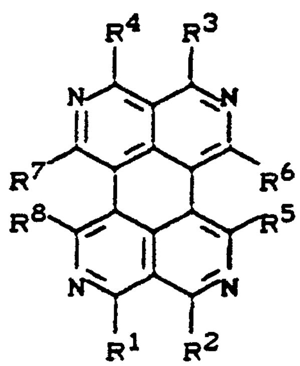

I,

worin

$R^1$ bis $R^8$ = H, Halogen, Alkyl, Cycloalkyl, Aralkyl, Aryl, heterocyclischer Rest, $NR^9R^{10}$, $OR^9$,

$SR^9$ wobei

$R^9$, $R^{10}$ = H, Alkyl, Cycloalkyl, Aralkyl, Aryl oder heterocyclischer Rest

wobei die Reste $R^1$, $R^2$ sowie $R^3$, $R^4$, weiterhin $R^5$, $R^6$ und $R^7$, $R^8$ zusammen Teile ankondensierter carbocyclischer oder heterocyclischer 5-, 6- oder 7-gliedriger Ringe bilden Können.

2. Verbindungen des Anspruchs 1 der Formel

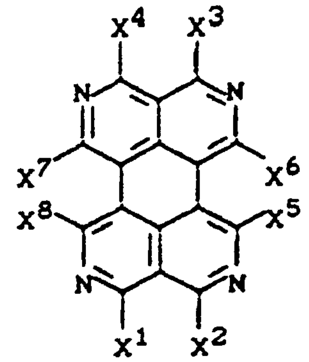

II,

worin $X^1$ bis $X^8$ gleich oder verschieden sein Können und für Br, Cl und F stehen.

3. Verbindung des Anspruchs 2 mit $X^1$ bis $X^8$ = Chlor.

4. Verbindungen des Anspruchs 1 der Formel

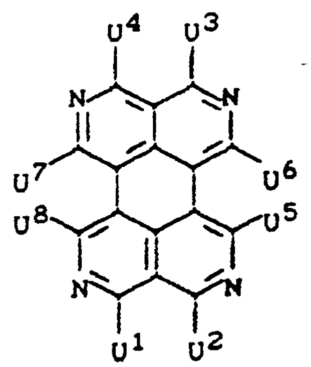

III,

worin $U^1$ bis $U^8$ gleich oder verschieden voneinander sein können und für $OR^9$, $SR^9R^{10}$ stehen.

5. Verbindungen des Anspruchs 1 der Formel

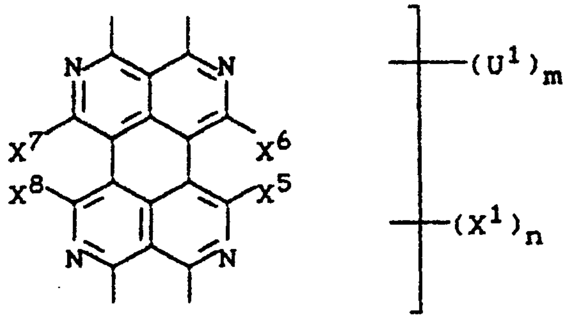

IV,

worin

$U^1$ für gleiche oder verschiedene Reste $OR^9$, $SR^9$, $NR^9R^{10}$ steht; $X^1$ bis $X^8$ gleich oder

verschieden sind und für F, Cl, Br stehen, und m und n = 1 - 4 bedeuten, wobei m + n = 4 sind.

6. Verbindungen des Anspruchs 5 der Formel

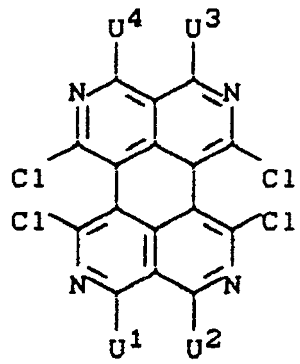

V,

worin $U^1$-$U^4$ wie in Anspruch 4 angegebene Bedeutung haben.

7. Verbindungen des Anspruchs 6 mit $U^1$-$U^4$ unabhängig voneinander = $Y^3$

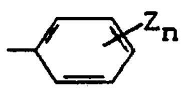

mit

$Y^3$ = 0, S, NH

n = 0, 1, 2, 3

Z = $OR^{11}$, $NR^{12}$, $R^{13}$, $SR^{11}$, $COOR^{11}$, CN, Br, Cl, F, $CONR^{12}R^{13}$, $SO_2R^{11}$, $SO_2OR^{11}$, $SO_2NR^{12}R^{13}$, -N=N-$R^{14}$, $R^{15}$, $OCOR^{11}$, $NR^{12}COR^{11}$, $COR^{11}$,

worin

$R^{11}$, $R^{12}$ und $R^{13}$ bezeichnen Wasserstoff, gegebenenfalls substituiertes Alkyl, insbesondere $C_1$-$C_{18}$-Alkyl, vorzugsweise $C_1$-$C_4$-Alkyl, gegebenenfalls substutiertes Cycloalkyl, insbesondere Cyclohexyl und Cyclopentyl, gegebenenenfalls substituiertes Aralkyl, insbesondere Phenyl- und Naphthyl-$C_1$-$C_4$-Alkyl, gegebenenfalls substituiertes Aryl, insbesondere Phenyl und Naphthyl, und einen gegebenenfalls substituiertes heterocyclischen Rest, insbesondere den Rest eines fünf- oder sechsgliedrigen heterocyclischen Ringes mit 1, 2 oder 3 Heteroatomen aus der Reihe O, N, S, an den ein Benzolring ankondensiert sein kann, wobei

$R^{12}$ und $R^{13}$ zusammen unter Einschluß des N-Atoms auch einen 5- oder 6-gliedrigen heterocyclischen Ring bilden können,

$R^{14}$ bezeichnet den Rest einer Kupplungskomponente, vorzugsweise einer Kupplungskomponente aus der Benzol-, Naphthalin-, Acetessigesterarylid-, Pyrazol- oder Pyridonreihe oder einen gegebenenfalls durch Cl, Br, F, $C_1$-$C_{18}$-Alkyl, vorzugsweise $C_1$-$C_4$-Alkyl und $C_1$-$C_{18}$-Alkoxy, vorzugsweise $C_1$-$C_4$-Alkoxy, substituierten Phenylrest,

$R^{15}$ bezeichnet gegebenenfalls substituiertes Alkyl, vorzugsweise $C_1$-$C_{18}$-Alkyl, insbesondere $C_1$-$C_4$-Alkyl und gegebenenfalls substituiertes Cycloalkyl, vorzugsweise $C_3$-$C_7$-Cycloalkyl, insbesondere Cyclohexyl und Cyclopentyl.

8. Verbindungen des Anspruchs 1 der Formel

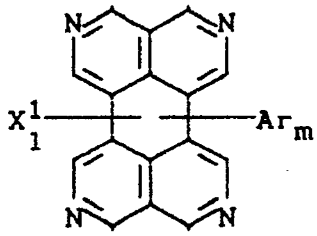

VI,

wobei

$X^1$ = F, Cl, Br und Ar = aromatischer oder heterocyclischer Rest, 1 und m = 0-8, wobei 1 + m = 8 und benachbarte Gruppen Ar auch Teile eines einzigen kondensierten aromatischen Systems sein können.

**9.** Verbindungen des Anspruchs 2 der Formel

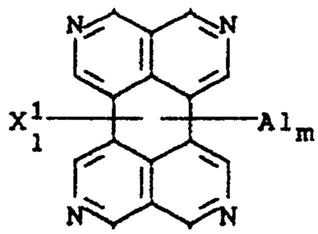

VII,

worin

$X_1$ = F, C1, Br und Al = gegebenfalls substituierter Alkyl-, Cycloalkyl- oder Aralkylrest, 1 und m = 0-8, wobei 1 + m = 8.

**10.** Verbindungen des Anspruchs 1 der Formel

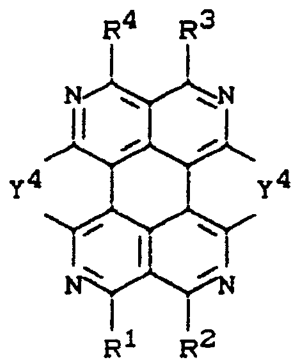

VIII,

worin $R^1$ bis $R^4$ die in Anspruch 1 angegebene Bedeutung haben,
und
$-Y^4-$ für -S-, -S-S-, -N=N-,

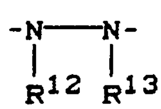 und 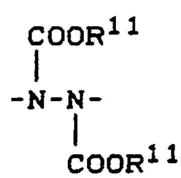

steht und $R^{11}$ bis $R^{14}$ die in Anspruch 7 angegebene Bedeutung haben.

**11.** Verbindungen des Anspruchs 10 der Formel

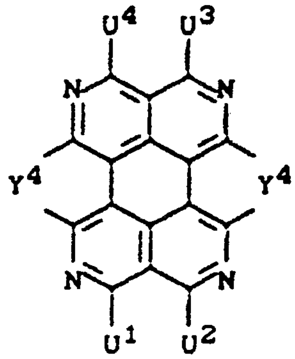

IX,

worin $U^1$-$U^4$ die in Anspruch 4 angegebene Bedeutung haben.

**12.** Verbindungen des Anspruchs 10 der Formel

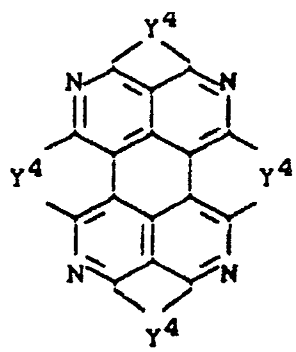

X,

wobei -$Y^4$- nicht Bestandteil eines Vierrings sein darf.

**13.** Verbindungen des Anspruchs 1 der Formel

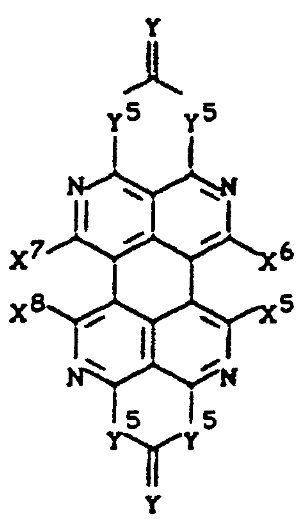

XI,

worin
$Y^5$ = O, $NR^{12}$, Y = O, S, $NR^{12}$, $NR^{13}$ und $X^5$-$X^8$ = F, Cl, Br.

**14.** Verbindungen des Anspruchs 13 mit $X^5$-$X^8$ = Cl und $Y^5$ = NH.

**15.** Verbindungen des Anspruchs 1 der Formel

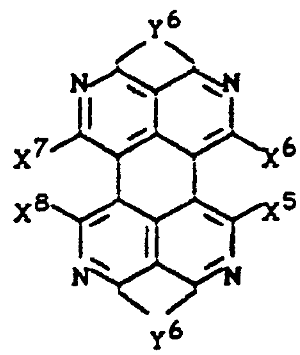

XIII,

worin $X^5$ bis $X^8$ = F, Cl, Br und $-Y^6-$ für die nachfolgenden Verbrückungen steht:

(a): 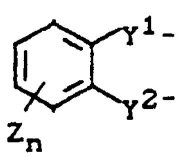 , (b): 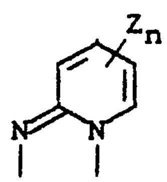 , (c): 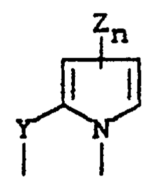

(d): 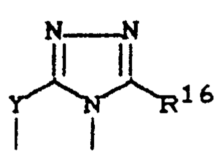 , (e): 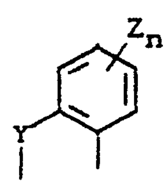 , (f): 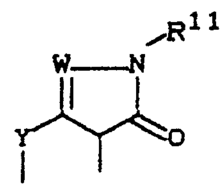

(g): 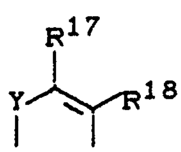 , (h): 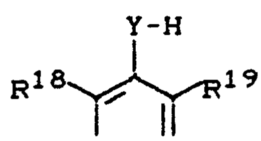 , (i): -S-S-

worin
W = N oder $C-R^{11}$,
Z, $R^{11}$-$R^{13}$ haben die in Anspruch 7 angegebene Bedeutung,
Y, $Y^1$, $Y^2$ = O, S, $NR^{12}$, $NR^{13}$,
$R^{16}$ = H oder Z
$R^{17}$ = gegebenenfalls substituiertes Aryl oder Hetaryl,
$R^{18}$ = CN, $COOR^{11}$, $COR^{11}$,
$R^{19}$ = CN, $COOR^{11}$, $COR^{11}$, $SO_2R^{11}$, $CONR^{12}R^{13}$.

**16.** Verbindungen des Anspruchs 15 der Formeln

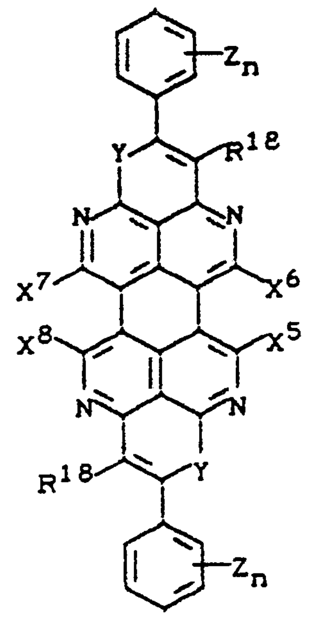

XIVa

bzw.

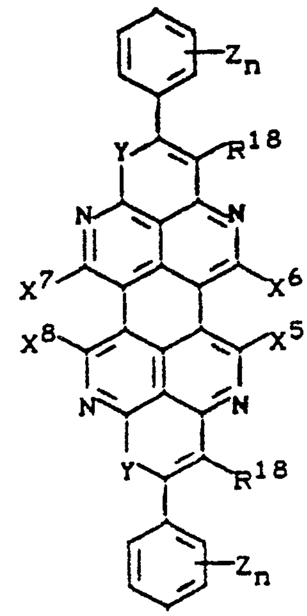

XIVb

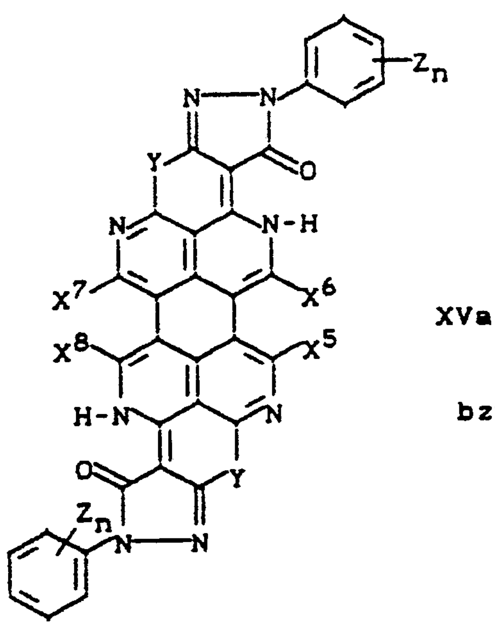

XVa

bzw.

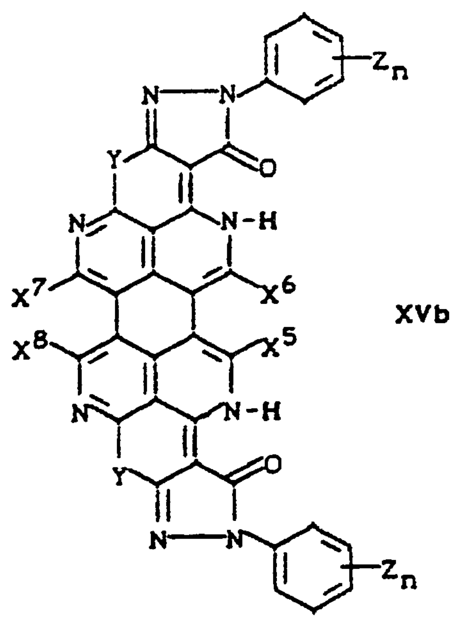

XVb

17. Verbindungen des Anspruchs 1 der Formel

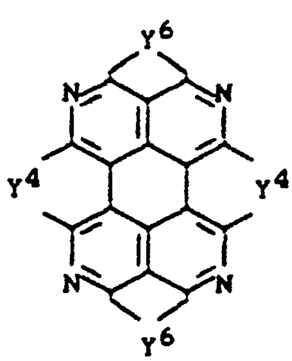

XVI,

worin
$Y^4$ die in Anspruch 10 angegebene Bedeutung hat und
$Y^5$ die in Anspruch 15 angegebene Bedeutung.

**18.** Verwendung der Verbindungen der Ansprüche 1-17 als Farbstoffe und Pigmente.